# EUROPÄISCHE PATENTSCHRIFT

(11) **EP 0 551 793 B1**
(45) Veröffentlichungstag und Bekanntmachung des Hinweises auf die Patenterteilung: **20.08.1997**
(21) Anmeldenummer: 92810958.6
(22) Anmeldetag: 04.12.1992
(51) Int. Cl.: A61F 2/38

(54) **Meniskusplattform zu einem künstlichen Kniegelenk**
Meniscus platform for artificial knee joint
Plate-forme ménisque pour articulation artificielle de genou

(30) Priorität: 14.01.1992 CH 90/92
(43) Veröffentlichungstag der Anmeldung: 21.07.1993
(73) Patentinhaber: Sulzer Orthopädie AG, 6340 Baar (CH)
(72) Erfinder: Wagner, Heinz, Prof. Dr. med., Orthop. Klinik, W-8501 Schwarzenbruck (DE); Willi, Roland, CH-8413 Neftenbach (CH)
(74) Vertreter: Heubeck, Bernhard

(56) Entgegenhaltungen:
- DE-A- 2 636 816
- FR-A- 2 663 536

## Beschreibung

Die Erfindung handelt von einer Meniskusplattform zu künstlichem Kniegelenk mit einer metallischen Plattform , welche auf ihrer Unterseite mit Verankerungselementen mit der Tibia verbunden ist, welche eine Aussparung für das hintere Kreuzband und stehengelassene Teile der Eminentia aufweist und welche einen Gleitkörper trägt, der auf seiner Oberseite Gleitflächen und einen Führungswulst für die Kondylen eines Femurteils aufweist, wobei der Gleitkörper und die metallische Plattform in einer Ebene senkrecht zur Tibiaachse zueinander gleitend sind, und wobei die Gleitbewegung einerseits durch einen auf der metallischen Plattform verankerten Führungszapfen und einem daran geführten Langloch im Gleitkörper begrenzt ist und andererseits durch eine begrenzende Aussparung im Gleitkörper eine durch die Eminentia im Bereich des hinteren Kreuzbandes beschränkte Schwenkbewegung um den Führungszapfen zugelassen wird.

Die Problematik und Lösungsformen für künstliche Kniegelenke sind ausführlich in US-PS 4,309,778 beschrieben. Die gezeigten Lösungsformen probieren den Bewegungsmechanismus des natürlichen Kniegelenks nachzuahmen und möglichst sicher in Bezug auf die Führung der beweglichen Elemente zu gestalten. Sie beanspruchen einen für die Sicherung der Führungsbewegung entsprechend grossen Raumanteil, um den eine Resektion im Knochengewebe des Tibiaknochens vorgenommen werden muss.

Die Patentanmeldung FR-A-26 63 536 offenbart eine Knieprothese mit einem Femurteil, der zwei doppelt gekrümmte Femurkondylen aus Metall aufweist, sowie ein metallisches Unterteil, welches nach der Durchführung eines Resektionsschnittes an der Tibia verankerbar ist und welches eine Gleitebene quer zur Tibiaachse aufweist. Es wird ein Zwischenstück zwischen Femurteil und Tibiateil vorgeschlagen, welches aus einem körperverträglichen Kunststoff besteht, welches um einen Stift in der Gleitebene drehbar gelagert ist, und welches zur Femurseite konkave Taschen aufweist, um eine Flexionsbewegung zwischen Femurkondylen und Zwischenstück sowie die Uebertragung einer Drehbewegung von den Femurkondylen auf das Zwischenstück zu ermöglichen. Eine solche Lösung bedingt eine relativ grosse Höhe vom Kunststoffzwischenstück, um den Festigkeitsanforderungen resp. einer geforderten Formbeständigkeit gerecht zu werden. Im weiteren entstehen zwischen Femurkondylen und Zwischenstück hohe Punktlasten, die zwar durch den elastischeren Kunststoff teilweise ausgeglichen werden aber gleichzeitig auch zu einem relativ hohen Verschleiss führen.

Die Offenlegungsschrift DE-A-26 36 816 offenbart ebenfalls eine Knieprothese mit einem als Tragschuh ausgebildeten Kunststoffkörper für doppelt gekrümmte Femurkondylen, welcher drehbar auf einem Tibiaunterteil gelagert ist. Auch hier müssen Drehbewegungen um die Tibiaachse über den Eingriff zwischen Femurkondylen und Kunststoffkörper übertragen werden und die Höhe des Resektionsschnittes wird durch die aus Festigkeitsgründen erforderliche Dicke des Kunststoffkörpers mitbestimmt.

Aufgabe der vorliegenden Erfindung ist es, den Anteil des abzutrennenden Knochenmaterials an der Tibia möglichst gering zu halten, ohne zu stark vom Bewegungsablauf des natürlichen Kniegelenks abzuweichen. Diese Aufgabe wird mit den kennzeichnenden Merkmalen von Anspruch 1 gelöst. Die Erfindung hat den Vorteil, dass durch den geringen Resektionsanteil das hintere Kreuzband und auch die Seitenbänder erhalten bleiben können und sich an der Wegbegrenzung für bewegliche Elemente ähnlich dem natürlichen Knie beteiligen und somit für die Führung der beweglichen Elemente untereinander weniger mechanische Anschläge notwendig sind. Die abhängigen Unteransprüche 2 und 3 beziehen sich auf vorteilhafte Weiterbildungen der Erfindung.

Im folgenden wird die Erfindung von einem Ausführungsbeispiel beschrieben. Es zeigen:

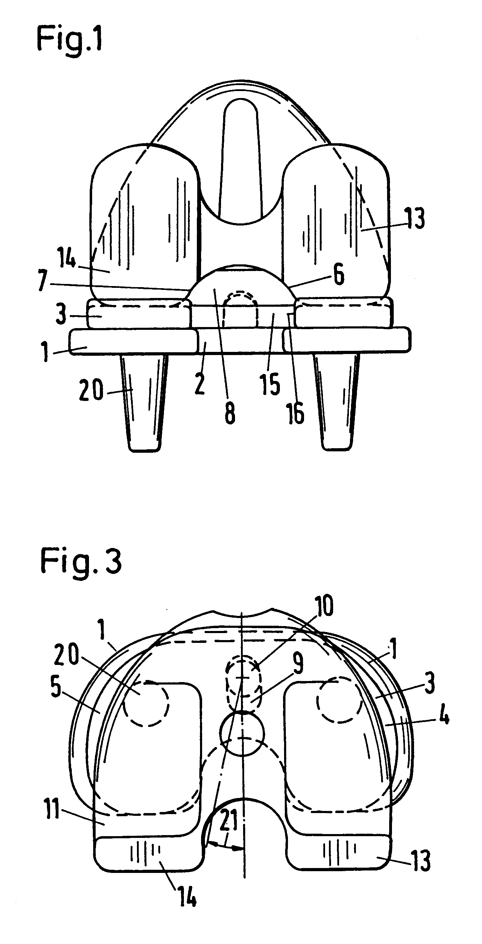

- Figur 1: Eine entsprechende Ansicht von posterior einer Kniegelenkprothese mit Tibiaplattform, Gleitkörper und Femurteil

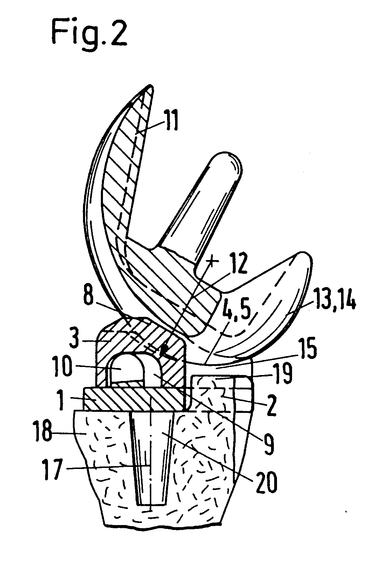

- Figur 2: eine schematische Draufsicht auf eine Prothese nach Figur 1; und
- Figur 3: schematisch die Seitenansicht eines Schnitts durch eine Prothese nach Figur 1 mit angedeuteter Tibia.

In den Figuren ist eine Meniskusplattform zu einem künstlichen Kniegelenk gezeigt, die aus einer metallischen Plattform 1 und einem quer zur Tibiaachse 17 darauf gleitenden metallischen Gleitkörper 3 besteht, der auf seiner Oberseite Gleitflächen 4, 5 und einen Führungswulst 8 für die Kondylen 13, 14 eines Femurteils 11 aufweist. Die Gleitbewegung zwischen metallischem Gleitkörper 3 und metallischer Plattform 1 ist dabei durch einen Führungszapfen 10 in der metallischen Plattform, der in ein Langloch 9 in einem Gleitkörper 3 aus Metall eingreift, begrenzt. Der metallische Gleitkörper 3 kann sich um den Führungszapfen 10 drehen und kann längs seinem Langloch 9 verschoben werden. Die Drehbewegung des Gleitkörpers 3 wird durch eine begrenzende Aussparung 15, die einen Teil der Eminentia 19 im Bereich des hinteren Kreuzbandes einschliesst, begrenzt.

Die metallische Plattform 1 ist mit zwei Verankerungselementen 20 in Form von Stiften in der Tibia 18 verankert und besitzt eine Aussparung 2, in die der im Bereich des hinteren Kreuzbandes stehengelassene Teil der Eminentia 19 hineinragt. In einer Ebene quer zur Tibiaachse 17 gleitet der Gleitkörper 3, der wie aus Figur 3 ersichtlich, kleinere Seitenabmessungen als die metallische Innenmass als die Aussparung 2 aufweist, um den Gleitkörper 3 aus der Mittellage mit dem daraufgeführten Femurteil 11 um einen Winkel 21 schwenken zu können, ohne dass der Gleitkörper 3 über die metallische Plattform 1 hinausragt. Als maximaler Schwenkwinkel ist ein Winkel 21 zwischen 5° und 20° vorgesehen. Die Seitenwangen 16 der begrenzenden Aussparung 15 sind deswegen zurückversetzt. Die Kondylen 13, 14 und die Gleitflächen 4, 5 gleiten auf gemeinsamen zylindrischen Flächen mit einem Radius 12. Der Führungswulst 8 liegt zwischen den beiden Kondylen 13, 14 und führt diese auf der Innenseite mit Seitenführungen 6, 7, die die gleiche Rotationsachse wie die zylindrischen Flächen der Kondylen 13, 14 aufweisen. Das Langloch 9 liegt bei ungeschwenktem Gleitkörper in sagittaler Richtung und besitzt eine Führungslänge von bis zu 8 mm für den Führungszapfen 10 in sagittaler Richtung.

## Patentansprüche

1. Meniskusplattform zu künstlichem Kniegelenk mit einer metallischen Plattform (1), welche auf ihrer Unterseite mit Verankerungselementen (20) mit der Tibia (18) verbunden ist, welche eine Aussparung (2) für das hintere Kreuzband und stehengelassene Teile der Eminentia (19) aufweist und welche einen Gleitkörper (3) trägt, der auf seiner Oberseite Gleitflächen (4, 5) und einen Führungswulst (8) für die Kondylen (13, 14) eines Femurteils (11) aufweist, wobei der Gleitkörper (3) und die metallische Plattform (1) in einer Ebene senkrecht zur Tibiaachse (17) zueinander gleitend sind, und wobei die Gleitbewegung einerseits durch einen auf der metallischen Plattform (1) verankerten Führungszapfen (10) und einem daran geführten Langloch (9) im Gleitkörper (3) begrenzt ist und andererseits durch eine begrenzende Aussparung (15) im Gleitkörper (3) eine durch die Eminentia im Bereich des hinteren Kreuzbandes beschränkte Schwenkbewegung um den Führungszapfen (10) zugelassen wird, dadurch gekennzeichnet, dass der Gleitkörper (3) aus Metall ist, dass Kondylen (13, 14) und Gleitflächen (4, 5) jeweils zylindrische Flächen mit einem konstanten Abrollradius (12) aufweisen, und dass die Aussparung (15) ein Ausschwenken aus der Mittellage bis zu einem Winkel (21) zulässt, ohne dass eine Ueberdeckung der Aussparung (2) stattfindet oder der Gleitkörper (3) über die Plattform (1) hinausragt.

2. Meniskusplattform nach Anspruch 1, dadurch gekennzeichnet, dass das Langloch (9) im nicht geschwenkten Gleitkörper (3) in sagittaler Richtung verläuft.

3. Meniskusplattform nach Anspruch 1 oder 2, dadurch gekennzeichnet, dass der Winkel (21) zwischen 5° und 20° beträgt.

## Claims

1. A meniscus platform for an artificial knee joint having a metallic platform (1), which on its under side is connected by attachment elements (20) to the tibia (18), which comprises a recess (2) for the rear cruciate ligament and untouched parts of the eminentia (19) and which supports a slide member (3), which on its upper side comprises sliding surfaces (4, 5) and a guide bead (8) for the condyles (13, 14) of a femoral part (11), with the slide member (3) and the metallic platform (1) being slidable in relation to one another in a plane perpendicular to the tibia axis (17), and with the sliding movement firstly being limited by a guiding pin (10) anchored on the metallic platform (1) and an oblong hole (9) guided thereon in the slide member (3) and secondly a swivelling moment, limited by the eminentia in the region of the rear cruciate ligament, around the guiding pin (10) being permitted by a limiting recess (15) in the slide member (3),
**characterised in that** the slide member (3) is made of metal,
**in that** condyles (13, 14) and sliding surfaces (4, 5) in each case comprise cylindrical faces having a constant rolling radius (12),
**and in that** the recess (15) permits a swivelling movement from the central position up to an angle (21) without a covering of the recess (2) occurring or the slide member (3) extending beyond the platform (1).

2. A meniscus platform according to Claim 1,
**characterised in that** the oblong hole (9) extends in the sagittal direction in the non-swivelled slide member (3).

3. A meniscus platform according to Claim 1 or 2,
**characterised in that** the angle (21) is between 5° and 20°.

## Revendications

1. Plate-forme ménisque pour articulation artificielle de genou avec une plate-forme métallique (1) qui est reliée à son côté inférieur par des éléments d'ancrage (20) au tibia (18), qui présente un évidement (2) pour le ligament croisé arrière et des parties qui subsistent de l'éminence (19) et qui porte un corps coulissant (3) qui présente sur son côté supérieur des faces coulissantes (4, 5) ainsi qu'un bourrelet de guidage (8) pour les condyles (13, 14) d'une partie de fémur (11), le corps coulissant (3) et la plate-forme métallique (1) pouvant coulisser l'un par rapport à l'autre dans un plan perpendiculairement à l'axe de tibia (17), et où le mouvement de coulissement est délimité d'une part par un tenon de guidage (10) ancré dans la plate-forme métallique (1) et un trou oblong (9) guidé à celui-ci dans le corps coulissant (3) et d'autre part, par un évidement de délimitation (15) dans le corps coulissant (3), un mouvement de pivotement limité par l'éminence au voisinage du ligament croisé arrière est admis autour du tenon de guidage (10),
caractérisée en ce que le corps coulissant (3) est en métal, que les condyles (13, 14) et les surfaces de glissement (4, 5) présentent respectivement des surfaces cylindriques avec un rayon de roulement constant (12), et que l'évidement (15) permet un pivotement depuis la position centrale jusqu'à un angle (21) sans qu'il y ait un recouvrement de l'évidement (2) ou que le corps coulissant (3) fasse saillie au-delà de la plate-forme (1).

2. Plate-forme ménisque selon la revendication 1,
caractérisée en ce que le trou oblong (9), à l'état non pivoté du corps coulissant (3), s'étend dans la direction sagittale.

3. Plate-forme ménisque selon la revendication 1 ou 2,
caractérisée en ce que l'angle (21) est compris entre 5° et 20°.
